(19) Europäisches Patentamt
European Patent Office
Office européen des brevets

(11) **EP 2 818 484 A1**

(12) # EUROPEAN PATENT APPLICATION

(43) Date of publication:
**31.12.2014 Bulletin 2015/01**

(51) Int Cl.:
***C07K 16/42*** (2006.01) ***A61K 31/557*** (2006.01)

(21) Application number: **13174325.4**

(22) Date of filing: **28.06.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Universitat Autònoma de Barcelona**
  **08193 Bellaterra (Cerdanyola del Valles) (ES)**
- **Universitat de Barcelona**
  **08028 Barcelone (ES)**
- **Hospital Clínic de Barcelona**
  **08036 Barcelona (ES)**

(72) Inventors:
- **Torres, Rosa**
  **08193 Cerdanyola del Vallès (ES)**
- **De Mora, Fernando**
  **08193 Cerdanyola del Vallès (ES)**
- **Picado, César**
  **08028 Barcelona (ES)**

(54) **Synergistic combination of an anti-IgE antibody and an EP2 receptor agonist**

(57) The present invention relates to synergistic combinations, including immunoconjugates, of an anti-IgE antibody, such as omalizumab, with an EP2 receptor agonist and their use in the prevention or treatment of an IgE-mediated disorder, preferably asthma.

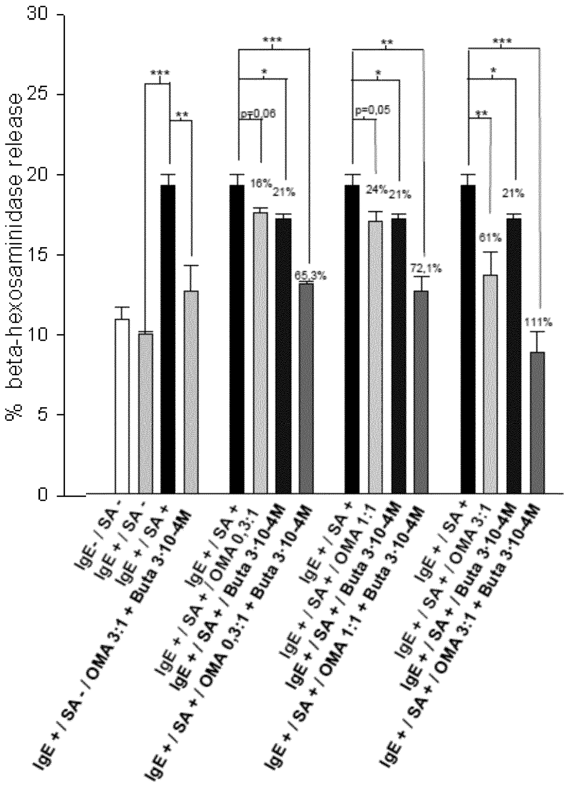


Figure 1

EP 2 818 484 A1

**Description**

## FIELD OF THE INVENTION

The present invention relates to a combination, including an immunoconjugate, of an anti-IgE antibody with an EP2 receptor agonist, and its use in the prevention or treatment of IgE-mediated disorders, preferably asthma.

## BACKGROUND OF THE INVENTION

IgE is a member of the immunoglobulin family that mediates type I hypersensitivity reactions. Type I reactions underlie at least in part the so-called allergic diseases to certain foods, drugs and environmental allergens.

Upon exposure to an allergen, the immune system produces IgE specific for that allergen. The resulting allergen-specific IgE then circulates through the bloodstream and binds to the IgE receptors located mainly on the surface of mast cells and basophils. Subsequent exposure to the allergen causes a cross linking of these IgE-receptor complexes which results in a release of a number of mediators such as histamine, prostaglandins, leukotrienes, proteases, cytokines, etc. from mast cells and basophils.

Treatment of IgE-mediated allergic reactions includes the administration of antihistamines, corticosteroids and other drugs which attempt to alleviate the symptoms associated with allergic reactions by counteracting the effects of the compounds released from mast cells and basophils. However, these drugs do not deal with the underlying cause of the disease and target effector mechanisms.

Another approach to the treatment of allergic disorders is the use of anti-IgE antibodies which recognize and bind circulating IgE and prevent it from binding to its receptor on effector cells, thereby preventing the release of mediators that result in pathological conditions. Currently marketed anti-IgE antibodies recognize and bind circulating IgE. However, for an anti-IgE antibody to be clinically useful it must be non-anaphylactogenic, i.e. it must not trigger the crosslinking of IgE-receptor complexes, since this would stimulate cellular activation and in turn would mimic the allergic response. Omalizumab (Xolair®) is an anti-IgE antibody approved for the treatment of IgE-mediated asthma and tested in other allergic and non-allergic conditions.

Selective and non-selective EP2-receptor agonists such as butaprost and $PGE_2$ have been found to inhibit activation of human lung mast cells (Kay et al., British Journal of Pharmacology 2006, 147, 707). In view of these results, it has been suggested that targeting EP2 receptors in the lung could prevent smooth muscle contraction and attenuate pulmonary inflammation. Despite the above, improved and more efficient treatments of allergic diseases are still needed.

## SUMMARY OF THE INVENTION

The inventors have established that EP2 receptor agonists potentiate the therapeutic effect of anti-IgE antibodies, thus providing a synergistic combination for the treatment of IgE-mediated disorders.

Therefore, in a first aspect the invention is directed to a combination comprising a non-anaphylactogenic anti-IgE antibody and an EP2 receptor agonist.

Another aspect of the invention refers to a combination as defined herein for use as a medicament.

In another aspect, the invention refers to a combination as defined herein for use in the prevention or treatment of an IgE-mediated disorder.

In a further aspect, the invention is directed to a pharmaceutical composition comprising a combination as defined herein and a pharmaceutically acceptable excipient.

Further aspects of the invention refer to the use of a combination as defined herein in the preparation of a medicament and in the preparation of a medicament for the prevention or treatment of an IgE-mediated disorder.

In another aspect, the invention refers to a method of preventing or treating an IgE-mediated disorder comprising administering to a subject in need of such treatment a non-anaphylactogenic anti-IgE antibody and an EP2 receptor agonist.

Another aspect of the invention refers to a kit comprising a dosage form of a non-anaphylactogenic anti-IgE antibody, a dosage form of an EP2 receptor agonist, and instructions for simultaneous, separate or sequential use thereof in the treatment of an IgE-mediated disorder.

In another aspect, the invention encompasses the use of an EP2 receptor agonist in potentiating the therapeutic effect of a non-anaphylactogenic anti-IgE antibody.

## BRIEF DESCRIPTION OF THE FIGURES

**Figure 1**. Graph showing the beta-hexosaminidase release (activation) from human mast cells (LAD2) after incubation with an anti-IgE antibody alone (omalizumab), an EP2 agonist alone (butaprost), and both compounds simultaneously. Percentage in the Y-axis represents the % inhibition caused by either the separate or the combined treatment. Graph represents mean±SEM of n=4 (OMA:omalizumab; Buta:butaprost; IgE-/SA-:resting cells; IgE+/SA-:sensitized but not activated cells (negative control); IgE+/SA+:activated cells; IgE+/SA+/Buta:butaprost treated activated cells; IgE+/SA+/OMA:omalizumab treated activated cells; IgE+/SA+/OMA+Buta: activated cells treated with omalizumab and butaprost; dose/concentration of omalizumab is expressed as the amount of omalizumab versus amount of IgE in culture).

## DETAILED DESCRIPTION OF THE INVENTION

The invention relates to synergistic combinations of non-anaphylactogenic anti-IgE antibodies and EP2 receptor agonists.

The two drugs may be part of the same composition (i.e. formulation) or may be provided as separate compositions.

In a particular embodiment, the non-anaphylactogenic anti-IgE antibody and the EP2 receptor agonist are part of the same composition.

In a another particular embodiment, the non-anaphylactogenic anti-IgE antibody as defined herein and the EP2 receptor agonist as defined herein are in the form of an immunoconjugate.

The term "immunoconjugate", as used herein, refers to an assembly formed by an EP2 receptor agonist linked or associated to an anti-IgE antibody that is therefore also acting as a carrier for the EP2 agonist to be released within the target environment.

As used herein, the term "synergistic" or "synergy" means that the effect achieved with the combination of a non-anaphylactogenic anti-IgE antibody and an EP2 receptor agonist is greater than the sum of the effects that result from using either the non-anaphylactogenic anti-IgE antibody or the EP2 receptor agonist as a monotherapy.

As used herein, the term "combination" means the combined administration of the non-anaphylactogenic anti-IgE antibody and the EP2 receptor agonist, which may be administered together in the same composition (i.e. a single product) or as separate compositions (i.e. separate products) administered simultaneously, sequentially or separately.

In a particular embodiment, the molar ratio of EP2 agonist to non-anaphylactogenic anti-IgE antibody in the combination of the invention is from 150000:1 to 1:1000, preferably from 10000:1 to 1:100 or from 1000:1 to 1:100, more preferably from 100:1 to 1:10 or from 10:1 to 1:10, even more preferably from 5:1 to 1:5, still more preferably from 3:1 to 1:3.

<u>$EP_2$ receptor agonists</u>

As used herein, the term "EP2 receptor agonist" or "EP2 agonist" refers to any compound that binds to the E prostanoid receptor 2 (EP2) resulting in adenylate cyclase stimulation. Any EP2 agonist may be used as the active compound of this invention.

EP2 agonists are well known for the skilled person in the art. Additionally, whether a compound is an EP2 agonist can be easily determined using, e,g. the Ready-to-Assay EP2 prostanoid receptor frozen cells (HTS185RTA, commercially available from Millipore).

EP2 agonists include, for example, butaprost, butaprost free acid from, ONO-8815, ONO-8815Ly, ONO-AE1-259, ONO-AE1-329, ONO-AE-248, ONO-4819, CP-533536, CAY10399, aganepag, aganepag isopropyl, taprenepag isopropyl, AS-02, AH13205, $PGE_1$, 11-deoxy-$PGE_1$, $PGE_2$, 16,16-dimethyl-$PGE_2$, 18-OH-$PGE_2$, 19-OH-$PGE_2$, 20-OH-$PGE_2$, 17-phenyl-ω-trinor-$PGE_2$, 11-deoxy-$PGE_2$-1-alcohol, misoprostol, misoprostol methyl ester, carbacyclin, isocarbacyclin, iloprost, cicaprost, MB 28767, $PGF_{2\alpha}$, $PGD_2$, U46619.

In a particular embodiment, the EP2 receptor agonist is selected from a compound of formula (I)

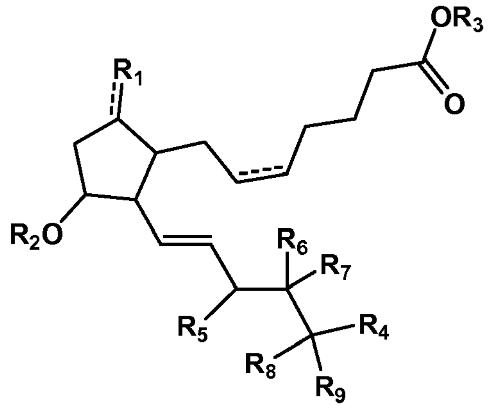

(I)

wherein

$R^1$ is selected from oxo, halogen and hydroxyl,
$R^2$ and $R^3$ are independently selected from hydrogen and $C_1$-$C_6$ alkyl,
$R^4$ is selected from $C_1$-$C_6$ alkyl optionally substituted by hydroxyl or $C_1$-$C_6$ alkoxyl; $C_2$-$C_6$ alkenyl and $C_6$-$C_{10}$ aryl,
$R^5$, $R^6$ and $R^7$ are independently selected from hydrogen, $C_1$-$C_6$ alkyl, hydroxyl and $C_1$-$C_6$ alkoxyl,
$R^8$ and $R^9$ are independently selected from hydrogen and $C_1$-$C_6$ alkyl, or $R^8$ and $R^9$ form together with the carbon atom to which they are attached a $C_3$-$C_6$ cycloalkyl, and ----- represents a single or a double bond,

or a pharmaceutically acceptable salt or solvate thereof.

In a particular embodiment, $R^1$ is selected from oxo and halogen, preferably from oxo and Cl.

In a particular embodiment, $R^2$ and $R^3$ are independently selected from hydrogen and $C_1$-$C_3$ alkyl, preferably from hydrogen and methyl. In an embodiment, $R^2$ is hydrogen and $R^3$ is selected from hydrogen and methyl.

In a particular embodiment, $R^4$ is selected from $C_1$-$C_3$ alkyl optionally substituted by hydroxyl; $C_2$-$C_4$ alkenyl and phenyl. Preferably, $R^4$ is selected from methyl, ethyl, propyl, 1-hydroxypropyl, 2-hydroxypropyl, 3-hydroxypropyl, 2-propenyl and phenyl.

In a particular embodiment, $R^5$, $R^6$ and $R^7$ are independently selected from hydrogen, $C_1$-$C_3$ alkyl, hydroxyl and $C_1$-$C_3$ alkoxyl. Preferably, from H, Me, OH and OMe. In a particular embodiment, $R^5$ is selected from H, OH and OMe, $R^6$ is selected from H, Me and OH and $R^7$ is selected from H, Me and OH.

In a particular embodiment, $R^8$ and $R^9$ are independently selected from hydrogen and C1-$C_3$ alkyl, or $R^8$ and $R^9$ form together with the carbon atom to which they are attached a $C_4$-$C_6$ cycloalkyl. Preferably, $R^8$ and $R^9$ are hydrogen or form, together with the carbon atom to which they are attached, a cyclobutyl.

In an embodiment, the EP2 receptor agonist is selected from a compound of formula (I) wherein:

$R^1$ is selected from oxo, Cl and OH,
$R^2$ and $R^3$ are independently selected from hydrogen and methyl,
$R^4$ is selected from methyl, ethyl, propyl, 1-hydroxypropyl, 2-hydroxypropyl, 3-hydroxypropyl, 2-propenyl and phenyl,
$R^5$, $R^6$ and $R^7$ are independently selected from H, Me, OH and OMe,
$R^8$ and $R^9$ are hydrogen or form, together with the carbon atom to which they are attached, a cyclobutyl, and ----- represents a single or a double bond,

or a pharmaceutically acceptable salt or solvate thereof.

Compounds of formula (I) are commercially available and/or can be prepared by standard synthetic methods well known for the skilled person. For example, compounds of formula (I) can be prepared following synthetic procedures disclosed in the art, e.g. in EP1008588 and EP0860430.

In a particular embodiment of the invention, compound of formula (I) is selected from butaprost, butaprost free acid form, ONO-8815, ONO-8815Ly, ONO-AE1-259, ONO-AE-248, CAY10399, $PGE_1$, $PGE_2$, 16,16-dimethyl-$PGE_2$, 18-OH-$PGE_2$, 19(R)-OH-$PGE_2$, 20-OH-$PGE_2$, 17-phenyl-ω-trinor-$PGE_2$, misoprostol, misoprostol methyl ester and $PGF_{2\alpha}$.

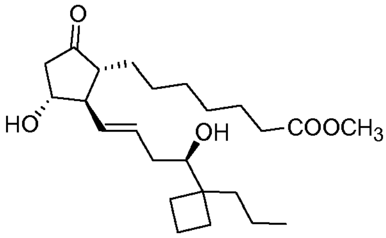

butaprost

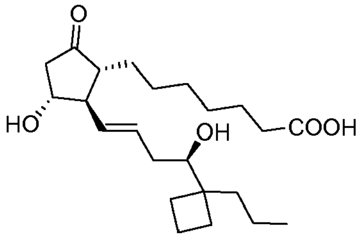

butaprost free acid form

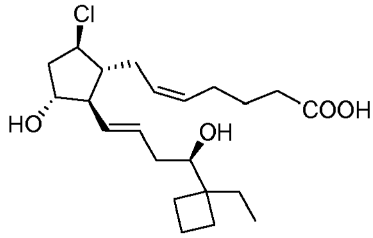

ONO-8815

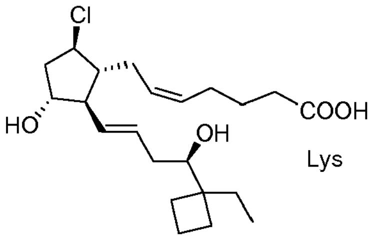

ONO-8815Ly

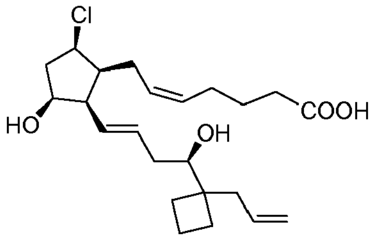

ONO-AE1-259

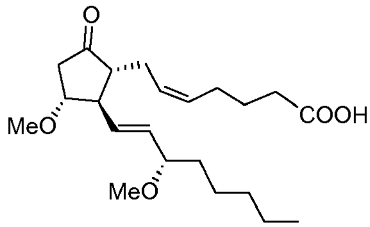

ONO-AE-248

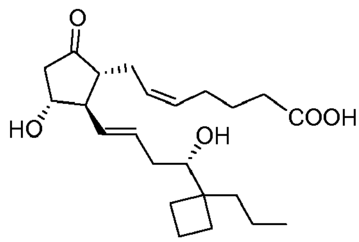

CAY10399

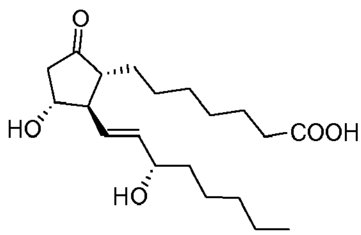

$PGE_1$

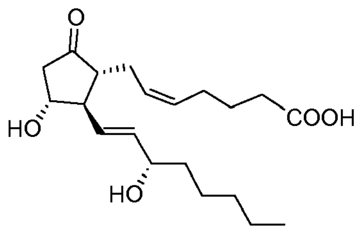

$PGE_2$

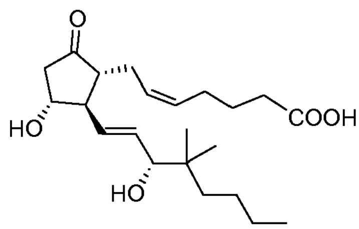

16,16-dimethyl-$PGE_2$

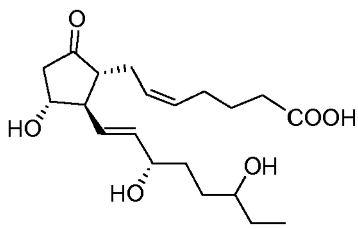

18-OH-$PGE_2$

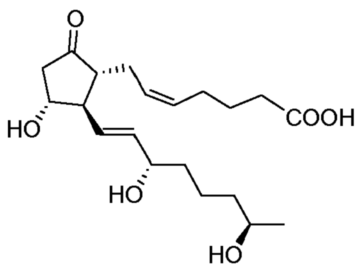

19(R)-OH-$PGE_2$

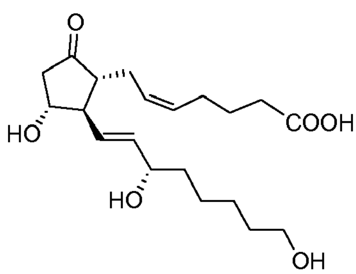

20-OH-$PGE_2$

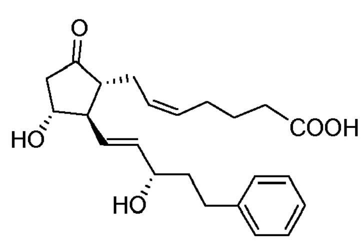

17-phenyl-ω-trinor-$PGE_2$

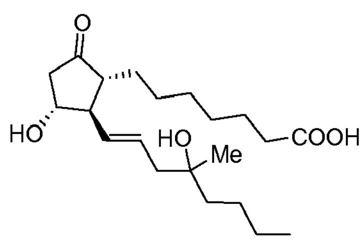

misoprostol

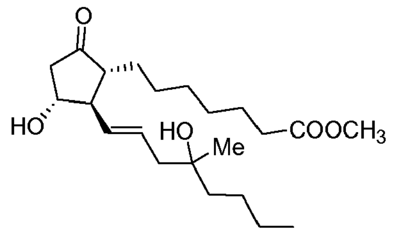

misoprostol methyl ester

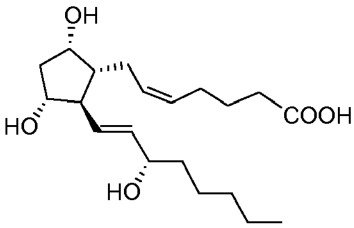

$PGF_{2\alpha}$

In an embodiment, EP2 receptor agonist is selected from butaprost, butaprost free acid form and CP-533536. In a particular embodiment, the EP2 receptor agonist is selected from butaprost and CP-533536. In another embodiment, the EP2 receptor agonist is butaprost.

As used herein, the term "$C_1$-$C_6$ alkyl" refers to a linear or branched alkane derivative containing from 1 to 6, preferably from 1 to 3 ("$C_1$-$C_3$ alkyl"), carbon atoms and which is bound to the rest of the molecule through a single bond. Illustrative examples of alkyl groups include methyl, ethyl, n-propyl, isopropyl, n-butyl, iso-butyl, tert-butyl, pentyl, hexyl, etc. Preferably, alkyl refers to methyl, ethyl or propyl. More preferably, alkyl is methyl.

The term "$C_3$-$C_6$ cycloalkyl" refers to a radical derived from cycloalkane containing from 3 to 6 carbon atoms. Illustrative examples of cycloalkyl groups include cyclopropyl, cyclobutyl, cyclopentyl, cyclohexyl. Preferably, cycloalkyl refers to cyclobutyl.

The term "alkenyl" refers to a linear or branched hydrocarbon chain having one or more double bonds therein. Suitable alkenyl groups include e.g. vinyl, allyl, butenyl, pentenyl, hexenyl, butadienyl. Preferably, alkenyl refers to allyl.

The term "alkoxyl" refers to an -O-alkyl group. Illustrative examples of alkoxyl groups include mehtoxy, ethoxy, propoxy, isopropoxy, butoxy and the like. Preferably, alkoxyl refers to methoxy.

The term "halogen" refers to bromine, chlorine, iodine or fluorine.

The term "aryl" refers to an aromatic group having between 6 and 10, preferably 6 or 10 carbon atoms, comprising 1 or 2 aromatic nuclei bound through a carbon-carbon bond or fused to one another. Illustrative examples of aryl groups include phenyl, naphthyl, biphenyl, etc. Preferably, aryl refers to phenyl.

The invention also provides "salts" of the compounds of formula (I). Preferably, said salts are base addition salts, which can be synthesized from the parent compounds containing an acid moiety by means of conventional chemical processes known by the persons skilled in the art. Such salts are generally prepared, for example, by reacting the free acid form of said compounds with a stoichiometric amount of the suitable base in water or in an organic solvent or in a mixture of the two. Non-aqueous media such as ether, ethyl acetate, ethanol, acetone, isopropanol or acetonitrile are generally preferred. Illustrative examples of base addition salts include inorganic base salts such as, for example, ammonium salts and organic base salts such as, for example, ethylenediamine, ethanolamine, *N,N*-dialkylenethanolamine, triethanolamine, glutamine or amino acid basic salts, such as lysine salt. In a particular embodiment, the base addition salt is an L-lysine salt.

Likewise, the compounds of formula (I) can be both as free compounds or as solvates (e.g., hydrates, alcoholates, etc.), both forms being included within the scope of the present invention. The solvation methods are generally known in the state of the art.

Anti-IgE antibody

As used herein, the term "anti-IgE antibody" refers to an antibody or a fragment of an antibody or a fusion protein (such as for instance *DARPin*-Fc), that binds to IgE molecules and neutralizes its activity. For instance it may neutralize its activity by inhibiting, or substantially reducing, the binding of the IgE molecules to the high affinity receptor FcεRI and/or the low affinity receptor FcεRII. In the case of the fusion protein, it may neutralize the activity by aggregating FcεRI-bound IgE with FcγRIIb on the surface of basophils.

Anti-IgE antibodies may bind and crosslink IgE on the surface of basophils and mast cells and trigger the release of the pharmacologic mediators of allergy; such antibody would be considered anaphylactogenic. Therefore, in order to be therapeutically useful, the anti-IgE antibody must be non-anaphylactogenic, i.e. it must either not bind to receptor-bound IgE or if it does, not trigger the cross-linkage, thereby avoiding cellular activation.

Non-anaphylactogenic anti-IgE antibodies are known in the art. Additionally, whether a compound is an anti-IgE antibody can be easily determined using, e,g. enzyme linked immunosorbent assay (ELISA), Western immunobloting, or other immunochemical techniques such as Surface Plasmon Resonance (Biacore). Non-anaphylactogenic anti-IgE activity can also be measured assessing the modulatory effect of anti-IgE on LAD2 human mast cells: The LAD2 human mast cell line expresses a functional FcεRI receptor for human IgE. Upon binding of 4(hydroxyl-3-nitrophenyl)acetyl (NP)-specific IgE to the LAD2 cells surface, and challenging with NP, the cells release β-hexosaminidase. The ability of

any anti-IgE antibody, or an antibody fragment, to prevent LAD2 cells sensitization by IgE, can be measured by activating the cells with NP (alternatively, biotinilated IgE and streptavidin can be used for activation) in the presence of the anti-IgE neutralizing antibodies. As a result of such neutralization LAD2 mast cells activity is inhibited as revealed by impaired β-hexosaminidase release.

The term "antibody" comprises monoclonal antibodies, or polyclonal antibodies, either intact or fragments thereof, and includes human, humanized and non-human antibodies.

The polyclonal antibodies are originally heterogeneous antibody molecule mixtures produced in the serum of animals which have been immunized with an antigen. They also include monospecific polyclonal antibodies obtained from heterogeneous mixtures, for example, by means of column chromatography with peptides of an individual epitope of the antigen of interest.

A monoclonal antibody is a homogenous population of specific antibodies for an individual epitope of the antigen. These monoclonal antibodies can be prepared by means of conventional techniques which have been described, for example, by the hybridoma method (Köhler et al., Nature 1975; 256: 495-497), or by recombinant methods (e.g. US4,816,567), or may be isolated from phage antibody libraries (Clackson et al., Nature 1991; 352:624-628; Marks et al., J. Mol. Biol. 1991; 222:581-597).

Human antibody is understood as an antibody integrally containing human light and heavy chains as well as constant regions, produced by means of any of the known standard methods. Humanized antibody is understood as an antibody from a non-human antibody, typically a murine antibody, which conserves the antigen binding properties of the parent antibody, but which is less immunogenic in human beings. This can be achieved by means of different processes, which include (a) grafting the complete nonhuman variable domains into human constant regions to generate chimeric antibodies; (b) grafting only the nonhuman complementarity determining regions (CDR) in a human framework and the constant regions, with or without retaining the critical framework residues; and (c) transplanting the complete nonhuman variable domains, but "concealing them" with a section similar to the human variable domain by means of replacing the surface residues.

The invention also comprises the use of fragments of the different types of antibodies mentioned above. The term "antibody fragment" includes, among others, antibody fragments such as Fab, $F(ab')_2$, Fab' and single chain Fv fragments (scFv).

Preferably, the anti-IgE antibody is a humanized murine antibody or a fully human antibody.

Preferably, the anti-IgE antibody is a monoclonal antibody.

Preferably, the anti-IgE antibody is a humanized monoclonal antibody.

Anti-IgE antibodies suitable for the present invention include, for example, omalizumab (also named E25, rhuMab25 or Xolair®), E26 (also named rhuMab26), E27 (also named rhuMab27), talizumab, CGP-51901, CGP-56901, BSW17, HAE1, HAE2, HMK-12 and 6HD5.

In a particular embodiment, the non-anaphylactogenic anti-IgE antibody is selected from omalizumab, BSW17, E26, E27 and talizumab. In an embodiment, the non-anaphylactogenic anti-IgE antibody is selected from omalizumab and BSW17. In another embodiment, the non-anaphylactogenic anti-IgE antibody is omalizumab.

In an embodiment of the invention, the EP2 agonist is selected from a compound of formula (I) as defined herein and the non-anaphylactogenic anti-IgE antibody is selected from omalizumab, E26, E27, talizumab, CGP-51901, CGP-56901, BSW17, HAE1, HAE2, HMK-12 and 6HD5. In a particular embodiment, the EP2 agonist is selected from a compound of formula (I) as defined herein and the non-anaphylactogenic anti-IgE antibody is selected from omalizumab and BSW17. In another embodiment, the EP2 agonist is selected from a compound of formula (I) as defined herein and the non-anaphylactogenic anti-IgE antibody is omalizumab.

In another embodiment, the EP2 agonist is selected from butaprost, butaprost free acid form, ONO-8815, ONO-8815Ly, ONO-AE1-259, ONO-AE-248, CP-533536, CAY10399, $PGE_1$, $PGE_2$, 16,16-dimethyl-$PGE_2$, 18-OH-$PGE_2$, 19-OH-$PGE_2$, 20-OH-$PGE_2$, 17-phenyl-ω-trinor-$PGE_2$, misoprostol, misoprostol methyl ester and $PGF_{2\alpha}$ and the non-anaphylactogenic anti-IgE antibody is selected from omalizumab, E26, E27, talizumab, CGP-51901, CGP-56901, BSW17, HAE1, HAE2, HMK-12 and 6HD5. In a particular embodiment, the EP2 agonist is selected from butaprost, butaprost free acid form, ONO-8815, ONO-8815Ly, ONO-AE1-259, CP-533536, CP-533536, CAY10399, $PGE_1$, $PGE_2$, 16,16-dimethyl-$PGE_2$, 18-OH-$PGE_2$, 19-OH-$PGE_2$, 20-OH-$PGE_2$, 17-phenyl-ω-trinor-$PGE_2$, misoprostol, misoprostol methyl ester and $PGF_{2\alpha}$ and the non-anaphylactogenic anti-IgE antibody is selected from omalizumab and BSW17, preferably is omalizumab.

In another embodiment, the EP2 agonist is butaprost and the non-anaphylactogenic anti-IgE antibody is selected from omalizumab, E26, E27, talizumab, CGP-51901, CGP-56901, BSW17, HAE1, HAE2, HMK-12 and 6HD5. In a particular embodiment, the EP2 agonist is butaprost and the non-anaphylactogenic anti-IgE antibody is selected from omalizumab, BSW17, E26, E27 and talizumab.

In an embodiment, the EP2 agonist is butaprost and the non-anaphylactogenic anti-IgE antibody is selected from omalizumab and BSW17. In another embodiment, the EP2 agonist is butaprost and the non-anaphylactogenic anti-IgE antibody is omalizumab.

In another embodiment, the EP2 agonist is CP-533536 and the non-anaphylactogenic anti-IgE antibody is selected from omalizumab, E26, E27, talizumab, CGP-51901, CGP-56901, BSW17, HAE1, HAE2, HMK-12 and 6HD5. In a particular embodiment, the EP2 agonist is CP-533536 and the non-anaphylactogenic anti-IgE antibody is selected from omalizumab, BSW17, E26, E27 and talizumab.

In an embodiment, the EP2 agonist is CP-533536 and the non-anaphylactogenic anti-IgE antibody is selected from omalizumab and BSW17. In another embodiment, the EP2 agonist is CP-533536 and the non-anaphylactogenic anti-IgE antibody is omalizumab.

IgE-mediated disorders

In an aspect, the invention is directed to a combination of an anti-IgE antibody and an EP2 receptor agonist as defined herein, for use as a medicament.

In another aspect, the invention is directed to a combination of an anti-IgE antibody and an EP2 receptor agonist as defined herein, for use in the prevention or treatment of IgE-mediated disorders.

In a particular embodiment, the non-anaphylactogenic anti-IgE antibody and the EP2 receptor agonist are in the form of an immunoconjugate.

The combinations and compositions of the present invention can be used to treat IgE-mediated disorders in a mammal, including humans or other mammals such as, for example, rodents, dogs, cats and horses.

The term "prevention" or "preventing" as used herein refers to the administration of a combination or composition of the invention either before a relapse occurs (e.g. in seasonal allergy) or in an initial or early stage of the disease, to avoid the appearance of clinical signs.

The term "treatment" or "treating" as used herein refers to the administration of a combination or composition of the invention to control the progression of the disease before or after clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total).

The term "IgE-mediated disorder" means a condition or disease which is characterized by the over-production of the immunoglobulin IgE. Specifically it should be construed to include conditions associated with anaphylactic hypersensitivity and allergies, including for example but not limited to asthma, systemic hypersensitivity, allergic rhinitis, atopic dermatitis, allergic contact dermatitis, urticaria, angioedema, allergic conjunctivitis, food allergy, drug allergy, respiratory allergy, allergic gastroenteropathy, allergic bronchopulmonary aspergillosis, allergic purpura.

In a particular embodiment, the IgE-mediated disorder is asthma.

The two drugs may be part of the same composition or may be administered simultaneously, separately or sequentially as separate compositions.

In a particular embodiment, the non-anaphylactogenic anti-IgE antibody and the EP2 receptor agonist of the combination of the invention are administered simultaneously, separately or sequentially.

As used herein, the term "simultaneously" means that the two therapeutic products (are administered concurrently under a single administration, "sequentially" means that the two therapeutic agents are available to act therapeutically within the same time-frame and "separately" means administration of the components of the combination independently of each other at different time points.

In a particular embodiment, the non-anaphylactogenic anti-IgE antibody and the EP2 receptor agonist are administered separately and at the same time or at different times. When administered separately and at different times, either the non-anaphylactogenic anti-IgE antibody or the EP2 receptor agonist may be administered first.

Pharmaceutical compositions

In another aspect, the invention is directed to a pharmaceutical composition comprising a combination as defined herein and a pharmaceutically acceptable excipient.

The term "pharmaceutically acceptable excipient" refers to a therapeutically inactive substance used for incorporating the active ingredient and which is acceptable for the patient from a pharmacological/toxicological point of view. The number and the nature of the pharmaceutically acceptable excipients depend on the desired dosage form. Such pharmaceutical excipients can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical excipients are described in "Remington's Pharmaceutical Sciences" by E.W. Martin, 21 st Edition, 2005. Preferably, the excipients of the invention are approved by a regulatory agency of the Federal or a state government or listed in the U.S. Pharmacopeia or other generally recognized pharmacopeia for use in animals,

and more particularly in humans.

The excipients and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galénica", C. Faulí i Trillo, Luzán 5, S.A. de Ediciones, 1993 or "Handbook of Pharmaceutical Excipients", Rowe C. R.; Paul J. S.; Marian E. Q., sixth Edition.

Examples of pharmaceutical compositions include any solid (tablets, pills, capsules, granules etc.) or liquid (solutions, suspensions or emulsions) compositions for oral, topical or parenteral administration.

The pharmaceutical compositions of the invention can be administered by any type of suitable route, such as by oral route, topical route, by inhalation or parenteral route so that the pharmaceutically acceptable excipients necessary for the formulation of the desired dosage form will be included.

In a particular embodiment, the pharmaceutical composition of the invention may be in a dosage form suitable for its administration by oral route, whether it is solid or liquid. Dosage forms suitable for their administration by oral route include tablets, capsules, syrups and solutions.

In another embodiment, the pharmaceutical composition of the invention may be in a dosage form suitable for its administration by inhalation. Dosage forms suitable for their administration by inhalation include for example aerosols, meter dosed inhalers, dry powder inhalers, aerosol jet and ultrasonic nebulizers.

"Topical route" is understood as an administration by non-systemic route, and includes the application of a pharmaceutical composition externally on the epidermis, in the oral cavity and the installation of said composition into ears, eyes and nose, and in which it does not significantly enter the blood stream.

"Inhalation" is understood as the administration by intranasal route and by oral inhalation. The dosage forms suitable for said administration, such as a formulation in aerosol, a meter dosed inhaler, a dry powder inhaler, an aerosol jet and an ultrasonic nebulizer can be prepared by means of conventional techniques.

As it is used herein, the term "parenteral", includes administration by intravenous route, intraperitoneal route, intramuscular route or subcutaneous route. Subcutaneous, intramuscular and intravenous dosage forms of parenteral administration are generally preferred.

The non-anaphylactogenic anti-IgE antibody and the $EP_2$ receptor agonist may be in one combined unit dosage or in two separate dosage forms.

Generally an effective administered amount of a compound of the invention will depend on the relative efficacy of the compound chosen, the severity of the disorder being treated and the weight of the sufferer. However, active compounds will typically be administered once or more times a day or a week, for example 1, 2, 3 or 4 times daily, or 1, 2, 3 or 4 times a week, or once every two weeks, or once every three weeks or once every four weeks. Typical total daily doses may be in the range of from 0.00001 to 10000 mg/kg/day, for example from 0.0001 to 100 mg/kg/day, preferably from 0.001 to 10 mg/kg/day, more preferably from 0.001 to 1 mg/kg/day, even more preferably from 0.01 to 1 mg/kg/day.

Another aspect of the invention refers to a kit comprising a dosage form of a non-anaphylactogenic anti-IgE antibody, a dosage form of an $EP_2$ receptor agonist, and instructions for simultaneous, separate or sequential use thereof in the treatment of an IgE-mediated disorder.

The following examples further illustrate the invention. These examples should not be interpreted as a limitation of the scope of the invention.

## EXAMPLES

**Example 1.** *Assessment of the synergic effect of the combination of an anti-IgE agent and an EP2 agonist in human mast cells.*

Cells: human LAD2 cell line expressing both the FcεRI (IgE high affinity receptor) and EP2 receptors were used.

Compounds tested: the anti-IgE monoclonal antibody omalizumab and the selective EP2 agonist butaprost.

Performance of the Assay:

- LAD2 cells were grown continuously in StemPro complete medium supplemented with stem cell factor (SCF).
- For the experiment, cells were resuspended in assay buffer (Hepes + 0.04%BSA).
- Before sensitizing cells, they were pre-incubated either with omalizumab (the following concentrations of omalizumab

to IgE were tried: 0.3:1, 1:1, 3:1, which correspond to 30 ng, 100 ng and 300 ng of omalizumab, respectively) and butaprost (36.6 μg) or with either one individually for 30 min and then 100 ng/ml Biotin-conjugated Human IgE was added for 2 hours.

- Cells activation was induced by adding 100 ng/ml of streptavidin (SA). After 30 min at 37°C, plate was centrifuged at 4°C and supernatants and pellets were transferred to a new plate in order to assay activation. Prior to this, cells were lysed using distilled water and frozen-thaw cycles.
- Incubation of supernatants and lysed cells with PNAG (p-nitrophenyl N-acetyl-b-D-glucosamide) lasted for 90 minutes at 37°C.
- Reaction was stopped using glycine and plate was read at 405nm with reference filter 620nm.

Statistical analysis:

Results are expressed as mean of % inhibition of beta-hexosaminidase release of n=4.

$$\% \text{ Inhibition} = 100 - (100 \times (\% \text{ net release of each condition} / \% \text{maximum release}))$$

$$\% \text{ beta-hexosaminidase release} = 100 \times (\text{supernatant content}) / (\text{supernatant} + \text{lysate content})$$

% Maximum release = % release in the sensitized and challenged cells
% Spontaneous release = % release in the sensitized non challenged cells
% Net release = % release of each condition-spontaneous release
Differences in % inhibition were assessed by Student's tests. Values of $p<0.05$ were considered significant.

Results obtained are shown in Figure 1. According to this assay, it was found that the combination of Omalizumab with Butaprost showed synergism in the inhibition of IgE-mediated activation of human mast cells (LAD2) at all the doses tried.

**Claims**

1. A combination comprising a non-anaphylactogenic anti-IgE antibody and an EP2 receptor agonist.

2. A combination according to claim 1, wherein the non-anaphylactogenic anti-IgE antibody and an EP2 receptor agonist are part of the same composition.

3. A combination according to claim 1, wherein the non-anaphylactogenic anti-IgE antibody and the EP2 receptor agonist are in the form of an immunoconjugate.

4. A combination according to any of claims 1 to 3, wherein the non-anaphylactogenic anti-IgE antibody is selected from omalizumab, E26, E27, talizumab, CGP-51901, CGP-56901, BSW17, HAE1, HAE2, HMK-12 and 6HD5.

5. A combination according to claim 4, wherein the non-anaphylactogenic anti-IgE antibody is omalizumab.

6. A combination according to any of claims 1 to 5, wherein the EP2 receptor agonist is selected from butaprost, butaprost free acid form, ONO-8815, ONO-8815Ly, ONO-AE1-259, ONO-AE1-329, ONO-AE-248, ONO-4819, CP-533536, CAY10399, aganepag, aganepag isopropyl, taprenepag isopropyl, AS-02, AH13205, $PGE_1$, 11-deoxy-$PGE_1$, $PGE_2$, 16,16-dimethyl-$PGE_2$, 18-OH-$PGE_2$, 19-OH-$PGE_2$, 20-OH-$PGE_2$, 17-phenyl-ω-trinor-$PGE_2$, 11-deoxy-$PGE_2$-1-alcohol, misoprostol, misoprostol methyl ester, carbacyclin, isocarbacyclin, iloprost, cicaprost, MB 28767, $PGF_{2\alpha}$, $PGD_2$ and U46619.

7. A combination according to any of claims 1 to 5, wherein the EP2 receptor agonist is selected from a compound of formula (I)

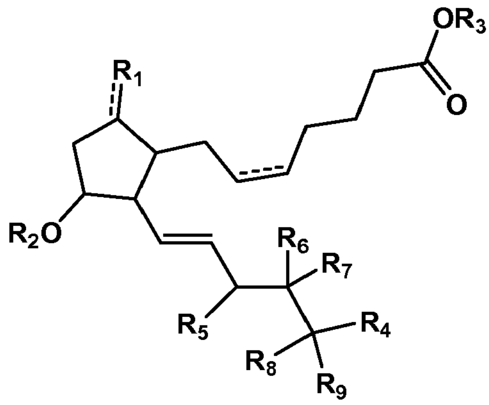

(I)

wherein
$R^1$ is selected from oxo, halogen and hydroxyl,
$R^2$ and $R^3$ are independently selected from hydrogen and $C_1$-$C_6$ alkyl,
$R^4$ is selected from $C_1$-$C_6$ alkyl optionally substituted by hydroxyl or $C_1$-$C_6$ alcoxyl; $C_1$-$C_6$ alkenyl and $C_6$-$C_{10}$ aryl,
$R^5$, $R^6$ and $R^7$ are independently selected from hydrogen, $C_1$-$C_6$ alkyl, hydroxyl and $C_1$-$C_6$ alkoxyl,
$R^8$ and $R^9$ are independently selected from hydrogen and $C_1$-$C_6$ alkyl, or $R^8$ and $R^9$ form together with the carbon atom to which they are attached a $C_3$-$C_6$ cycloalkyl, and
----- represents a single or a double bond,
or a pharmaceutically acceptable salt or solvate thereof.

**8.** A combination according to any of claims 1 to 7, wherein the EP2 receptor agonist is selected from butaprost and CP-533536.

**9.** A combination according to any of claims 1 to 8, wherein the non-anaphylactogenic anti-IgE antibody is omalizumab and the EP2 receptor agonist is butaprost.

**10.** A combination according to any of claims 1 to 9 for use as a medicament.

**11.** A combination according to any of claims 1 to 9 for use in the prevention or treatment of an IgE-mediated disorder.

**12.** A combination for use according to claim 11, wherein the IgE-mediated disorder is selected from asthma, systemic hypersensitivity, allergic rhinitis, atopic dermatitis, allergic contact dermatitis, urticaria, angioedema, allergic conjunctivitis, food allergy, drug allergy, respiratory allergy, allergic gastroenteropathy, allergic bronchopulmonary aspergillosis, allergic purpura.

**13.** A combination for use according to claim 12, wherein the IgE-mediated disorder is asthma.

**14.** A combination for use according to any of claims 10 to 13, wherein the non-anaphylactogenic anti-IgE antibody and the EP2 receptor agonist are administered simultaneously, separately or sequentially.

**15.** A pharmaceutical composition comprising a combination according to any of claims 1 to 9 and a pharmaceutically acceptable excipient.

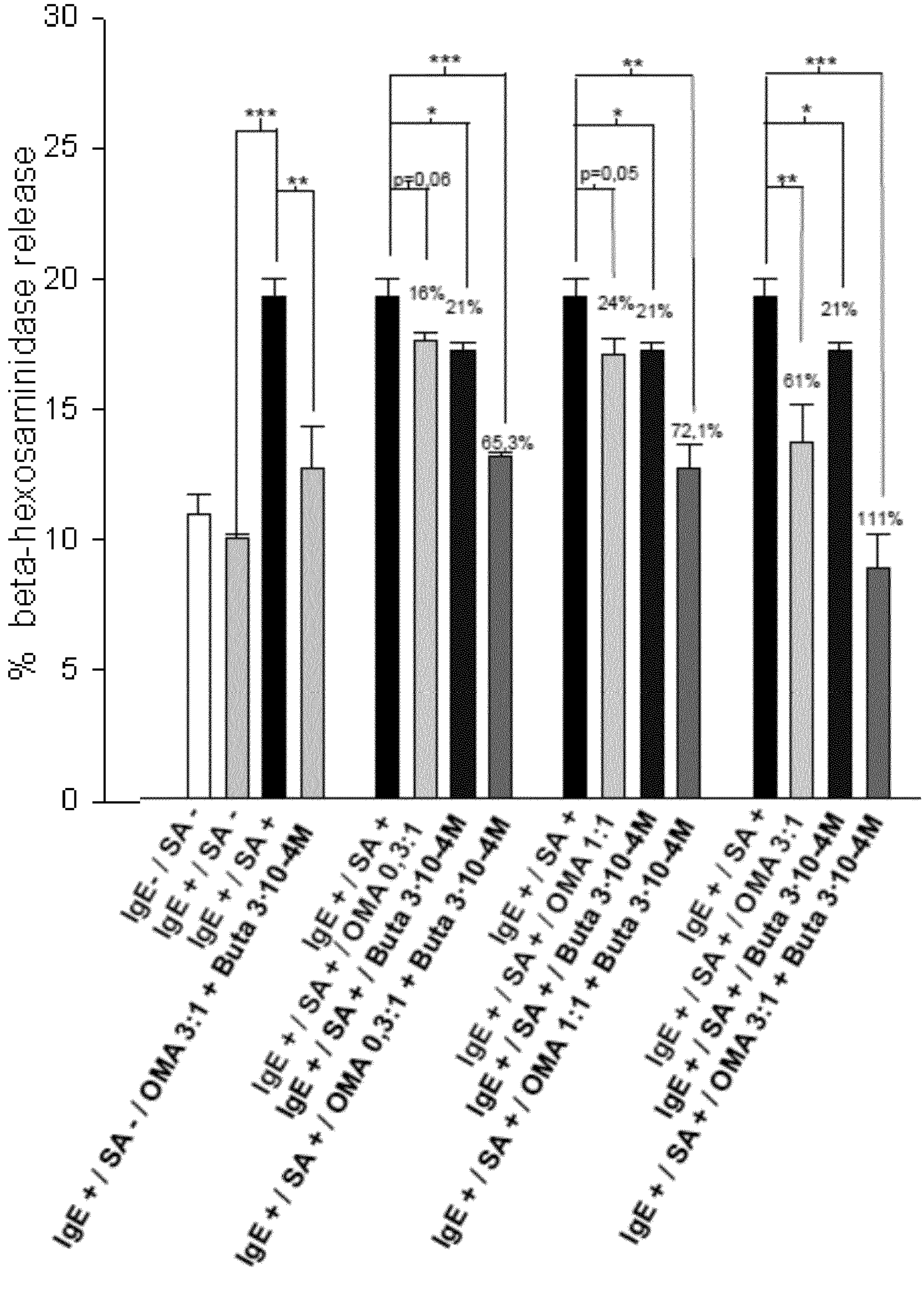
% beta-hexosaminidase release
30
25
20
15
10
5
0
***
**
***
*
p=0,06
16%
21%
65,3%
**
*
p=0,05
24%
21%
72,1%
***
*
**
21%
61%
111%
IgE- / SA -
IgE + / SA -
IgE + / SA +
IgE + / SA - / OMA 3:1 + Buta 3·10-4M
IgE + / SA +
IgE + / SA + / OMA 0,3:1
IgE + / SA + / Buta 3·10-4M
IgE + / SA + / OMA 0,3:1 + Buta 3·10-4M
IgE + / SA +
IgE + / SA + / OMA 1:1
IgE + / SA + / Buta 3·10-4M
IgE + / SA + / OMA 1:1 + Buta 3·10-4M
IgE + / SA +
IgE + / SA + / OMA 3:1
IgE + / SA + / Buta 3·10-4M
IgE + / SA + / OMA 3:1 + Buta 3·10-4M

Figure 1

Europäisches Patentamt
European Patent Office
Office européen des brevets

# EUROPEAN SEARCH REPORT

Application Number
EP 13 17 4325

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SERRA-PAGES MARIONA ET AL: "E-prostanoid 2 receptors dampen mast cell degranulation via cAMP/PKA-mediated suppression of IgE-dependent signaling", JOURNAL OF LEUKOCYTE BIOLOGY,, vol. 92, no. 6, 1 December 2012 (2012-12-01), pages 1155-1165, XP009174133, ISSN: 1938-3673 * the whole document * ----- | 1-15 | INV. C07K16/42 A61K31/557 |
| A | LINDA J KAY ET AL: "Prostaglandin E 2 activates EP 2 receptors to inhibit human lung mast cell degranulation", BRITISH JOURNAL OF PHARMACOLOGY, vol. 147, no. 7, 1 April 2006 (2006-04-01), pages 707-713, XP055088436, ISSN: 0007-1188, DOI: 10.1038/sj.bjp.0706664 * the whole document * ----- | 1-15 | |
| A | L. J. KAY ET AL: "Characterization of the EP receptor subtype that mediates the inhibitory effects of prostaglandin E 2 on IgE-dependent secretion from human lung mast cells", CLINICAL & EXPERIMENTAL ALLERGY, vol. 43, no. 7, 21 June 2013 (2013-06-21), pages 741-751, XP055088439, ISSN: 0954-7894, DOI: 10.1111/cea.12142 * abstract * ----- -/-- | 1-15 | TECHNICAL FIELDS SEARCHED (IPC) C07K A61K |

The present search report has been drawn up for all claims

1

| Place of search | Date of completion of the search | Examiner |
|---|---|---|
| Munich | 14 November 2013 | Kalsner, Inge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 4325

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VENNERA MARÍA DEL CARMEN ET AL: "Omalizumab therapy in severe asthma: experience from the Spanish registry--some new approaches", THE JOURNAL OF ASTHMA,, vol. 49, no. 4, 1 May 2012 (2012-05-01), pages 416-422, XP009174134, * abstract * ----- | 1-15 | |

TECHNICAL FIELDS SEARCHED (IPC)

The present search report has been drawn up for all claims

1

| Place of search | Date of completion of the search | Examiner |
|---|---|---|
| Munich | 14 November 2013 | Kalsner, Inge |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1008588 A
- EP 0860430 A
- US 4816567 A


### Non-patent literature cited in the description


- **KAY et al.** *British Journal of Pharmacology,* 2006, vol. 147, 707
- **KÖHLER et al.** *Nature,* 1975, vol. 256, 495-497
- **CLACKSON et al.** *Nature,* 1991, vol. 352, 624-628
- **MARKS et al.** *J. Mol. Biol.,* 1991, vol. 222, 581-597
- **E.W. MARTIN.** Remington's Pharmaceutical Sciences. 2005
- **C. FAULÍ I TRILLO.** Tratado de Farmacia Galénica. 1993
- **ROWE C. R. ; PAUL J. S. ; MARIAN E. Q.** Handbook of Pharmaceutical Excipients